# EUROPÄISCHE PATENTSCHRIFT

(11) **EP 3 173 841 B1**
(45) Veröffentlichungstag und Bekanntmachung des Hinweises auf die Patenterteilung: **01.01.2020**
(21) Anmeldenummer: 16197009.0
(22) Anmeldetag: 03.11.2016
(51) Int. Cl.: G02B 23/24, A61B 1/002, G02B 13/18, G02B 27/00

(54) **STABLINSENSYSTEM FÜR EIN ENDOSKOP SOWIE ENDOSKOP MIT EINEM SOLCHEN STABLINSENSYSTEM**
ROD LENS SYSTEM FOR AN ENDOSCOPE, AND ENDOSCOPE COMPRISING THE ROD LENS SYSTEM
SYSTÈME DE LENTILLE EN FORME DE BARRE POUR UN ENDOSCOPE ET ENDOSCOPE COMPRENANT UN TEL SYSTÈME DE LENTILLE EN FORME DE BARRE

(30) Priorität: 13.11.2015 DE 102015119622
(43) Veröffentlichungstag der Anmeldung: 31.05.2017
(73) Patentinhaber: Henke-Sass, Wolf GmbH, 78532 Tuttlingen (DE)
(72) Erfinder: REHE, Oliver, 78573 Wurmlingen (DE)
(74) Vertreter: Patentanwälte Geyer, Fehners & Partner mbB

(56) Entgegenhaltungen:
- DE-T5-112004 002 177
- US-A- 5 632 718
- US-A- 6 088 157
- US-A1- 2008 273 247
- US-A1- 2015 256 721

## Beschreibung

Die vorliegende Erfindung betrifft ein Stablinsensystem für ein Endoskop mit den Merkmalen des Oberbegriffs des Anspruches 1. Des Weiteren betrifft sie ein Endoskop mit einem solchen Stablinsensystem.

Das Stablinsensystem wird bei Endoskopen dazu genutzt, um ein in einer distalen Zwischenbildebene liegendes Zwischenbild eines mittels des Endoskops zu betrachtenden Objektes in eine proximale Zwischenbildebene abzubilden und somit das Zwischenbild weiterzuleiten.

Solche Stablinsensysteme weisen in der Regel sich wiederholende Stablinsen in symmetrischen Umkehrstufen auf, um die Kosten des Gesamtsystems niedrig zu halten. Dabei sind identische Stablinsen gegensinnig eingebaut. Dies führt zu der Schwierigkeit, dass ein Farblängsfehler, der durch das Stablinsensystem bedingt ist, nur schwer korrigierbar ist.

Aus der US 6 088 157 A, der US 2015/256721 A1 und der US 2008/273247 A1 ist jeweils ein Stablinsensystem mit den Merkmalen des Oberbegriffs des Anspruches 1 bekannt.

Ausgehend hiervon ist es Aufgabe der Erfindung, ein Stablinsensystem für ein Endoskop bereitzustellen, das die eingangs genannten Schwierigkeiten verringert und möglichst vollständig überwindet. Ferner soll ein Endoskop mit einem solchen Stablinsensystem bereitgestellt werden.

Die Erfindung ist im Anspruch 1 und im Anspruch 14 definiert. Vorteilhafte Ausgestaltungen sind in den abhängigen Ansprüchen angegeben.

Beim erfindungsgemäßen Stablinsensystem ist es möglich, mehrere Gläser und Geometrien (Radien und Dicken) bei den Stablinsen vorzusehen, wodurch eine bessere Korrektur des Farblängsfehlers möglich ist.

Jede Umkehrstufe ist bevorzugt so ausgebildet, dass sie ein Zwischenbild in eine nächste Zwischenbildebene abbildet. Wenn mehrere hintereinander angeordnete Umkehrstufen vorgesehen sind, sind diese bevorzugt so angeordnet, dass die nächste Zwischenbildebene einer Umkehrstufe mit der Zwischenbildebene zusammenfällt, aus der die nachfolgende Umkehrstufe ein Zwischenbild in die nächste Zwischenbildebene dieser nachfolgenden Umkehrstufe abbildet.

Insbesondere ist die Umkehrstufe bzw. sind die Umkehrstufen des Stablinsensystems so angeordnet, dass die Zwischenbildebene, aus der die am nähesten an der distalen Zwischenbildebene liegende Umkehrstufe das Zwischenbild in die nächste Zwischenbildebene abbildet, mit der distalen Zwischenbildebene zusammenfällt. Des Weiteren kann die nächste Zwischenbildebene der Umkehrstufe, die am nähesten an der proximalen Zwischenbildebene liegt, mit der proximalen Zwischenbildebene zusammenfallen.

Bevorzugt bildet jede Umkehrstufe das Zwischenbild in die nächste Zwischenbildebene umgekehrt oder auf dem Kopf stehend ab. Es ist jedoch auch eine aufrechte Abbildung möglich.

Bei dem erfindungsgemäßen Stablinsensystem können zwei Umkehrstufen als unsymmetrische Umkehrstufen ausgebildet sein. Insbesondere können alle Umkehrstufen als unsymmetrische Umkehrstufen ausgebildet sein.

Ferner kann eine erste Umkehrstufe, die am nächsten an der distalen Zwischenbildebene liegt, einen Abbildungsmaßstab von größer als 1 aufweisen. Eine zweite Umkehrstufe, die am nächsten zur ersten Umkehrstufe liegt, kann einen Abbildungsmaßstab von kleiner als 1 aufweisen. Insbesondere können die erste und zweite Umkehrstufe zusammen einen Abbildungsmaßstab von 1 aufweisen.

Ferner kann eine der Umkehrstufen eine einer der Zwischenbildebenen zugewandte gekrümmte Grenzfläche aufweisen, die asphärisch gekrümmt ist. Die asphärische Krümmung kann dabei eine Rotationssymmetrie aufweisen. Es ist jedoch auch möglich, dass die asphärische Krümmung keine Rotationssymmetrie aufweist und in den zwei Hauptschnitten unterschiedlich gekrümmt ist.

Bevorzugt weist mindestens eine Stablinse jedes Umkehrsystems mindestens eine gekrümmte (z. B. sphärisch oder asphärisch gekrümmt) Materialgrenzfläche auf.

Bei dem erfindungsgemäßen Stablinsensystem können die Umkehrstufen jeweils zumindest zwei Stablinsen aufweisen, wobei jede der Umkehrstufen zwei verschiedene Stablinsentypen aufweist. Unter einem Stablinsentyp wird insbesondere verstanden, dass Stablinsen des gleichen Typs gleich ausgebildet sind. Sie weisen also gleiche Materialien, gleiche Abmessungen und gleiche Krümmungen der Materialgrenzflächen auf. Sie können in den Umkehrsystemen relativ zueinander gleichsinnig oder gegensinnig angeordnet sein.

Beim erfindungsgemäßen Stablinsensystem unterscheiden sich die Stablinsen des ersten, zweiten und dritten Typs. Damit ist eine ausgezeichnete Korrektur des Farblängsfehlers möglich.

Ferner kann zumindest eine der Stablinsen aus zumindest zwei Teilen aufgebaut sein. Insbesondere kann sie als Kittglied ausgebildet sein. Ferner ist es möglich, dass zumindest eine der Stablinsen einstückig ausgebildet ist.

Das erfindungsgemäße Stablinsensystem ist insbesondere für starre Endoskope oder Endoskope mit starrem Endoskopschaft vorgesehen.

Die Stablinsen der Umkehrsysteme können einen Durchmesser im Bereich von 1 bis 6,5 mm und insbesondere im Bereich von 1,7 bis 5 mm aufweisen. Die Länge einer Umkehrstufe kann im Bereich von 30 bis 120 mm liegen. Insbesondere ist eine Länge im Bereich von 40 bis 80 mm oder eine Länge von 60 mm bevorzugt.

Die Anzahl der Umkehrstufen des Stablinsensystems kann im Bereich von eins bis elf oder zwei bis elf Umkehrstufen liegen. Bevorzugt ist eine ungerade Anzahl von Umkehrstufen. Somit sind insbesondere eine Umkehrstufe, drei, fünf, sieben, neun und elf Umkehrstufen möglich. Natürlich ist es auch möglich, eine gerade Anzahl von Umkehrstufen vorzusehen.

Das Stablinsensystem kann insbesondere einen Abbildungsmaßstab im Bereich von 0,5 bis 2 aufweisen. Größere oder kleinere Werte sind ebenfalls möglich.

Die Stablinsen können aus unterschiedlichen Materialien gebildet sein. Insbesondere kann eine einzelne Stablinse selbst aus zwei unterschiedlichen Materialien gebildet sein. Dabei sind insbesondere Glas- und Kunststoffmaterialien bevorzugt. Damit ist eine gute Farbfehlerkorrektur möglich.

Das Stablinsensystem kann so ausgebildet sein, dass in der proximalen Zwischenbildebene die Hauptstrahlen bei der Abbildung divergent verlaufen. Dies führt zu einem größeren Abstand der Austrittspupille entlang der optischen Achse des Stablinsensystems. Alternativ ist es möglich, dass die Hauptstrahlen konvergent verlaufen, was zu einem geringeren Abstand der Austrittspupille führt. Damit ist eine bessere Anpassung an z. B. vorzusehende Kamerasysteme zur Aufnahme des Zwischenbildes aus der proximalen Zwischenbildebene möglich.

Die Verwendung von einer oder mehrerer asphärischen Materialgrenzflächen bei den Stablinsen kann insbesondere zu einer Verringerung der Vignettierung des optischen Gesamtsystems und/oder zu einer Verringerung der asphärischen Aberration des Gesamtsystems genutzt werden. Eine verringerte Vignettierung führt zu einer höheren Randhelligkeit und eine Verringerung der asphärischen Aberration führt zu einer höheren Randschärfe.

Mittels des erfindungsgemäßen Stablinsensystems kann die Bildfeldkrümmung korrigiert werden, was zu einer Bildschale führt, die eine größere Ebenheit aufweist, wodurch eine höhere Randschärfe bei der Abbildung erzielt wird. Des Weiteren kann der Astigmatismus korrigiert werden, wodurch wiederum die Randschärfe bei der Abbildung verbessert werden kann.

Die Stablinsen zumindest einer Umkehrstufe und/oder die Umkehrstufen weisen bevorzugt eine gemeinsame optische Achse auf.

Ferner wird ein Endoskop mit einem erfindungsgemäßen Stablinsensystem (einschließlich der Weiterbildung des erfindungsgemäßen Stablinsensystems) bereitgestellt. Das Endoskop kann ein Objektiv aufweisen, das dem Stablinsensystem vorgeordnet ist. Insbesondere ist das Objektiv so ausgebildet, dass es ein abzubildendes Objekt in die distale Zwischenbildebene abbildet.

Des Weiteren kann das Endoskop eine dem Stablinsensystem nachgeordnete Optikeinheit aufweisen, wie z. B. ein Okular.

Das erfindungsgemäße Endoskop kann weitere dem Fachmann bekannte Merkmale aufweisen, die zum Betrieb des Endoskops notwendig sind.

Insbesondere kann das Endoskop als Endoskop mit einem starren Schaft ausgebildet sein, in dem das erfindungsgemäße Stablinsensystem angeordnet ist.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung beispielsweise anhand der beigefügten Zeichnungen, die auch erfindungswesentliche Merkmale offenbaren, noch näher erläutert. Es zeigen:
- Fig. 1: eine schematische Darstellung des erfindungsgemäßen Stablinsensystems 1 in einem Endoskop 2;
- Fig. 2: eine schematische Darstellung des optischen Gesamtsystems des Endoskops 2 von Fig. 1, und
- Fig. 3: eine vergrößerte Ansicht der Stablinse 21 des dritten Umkehrsystems 12.

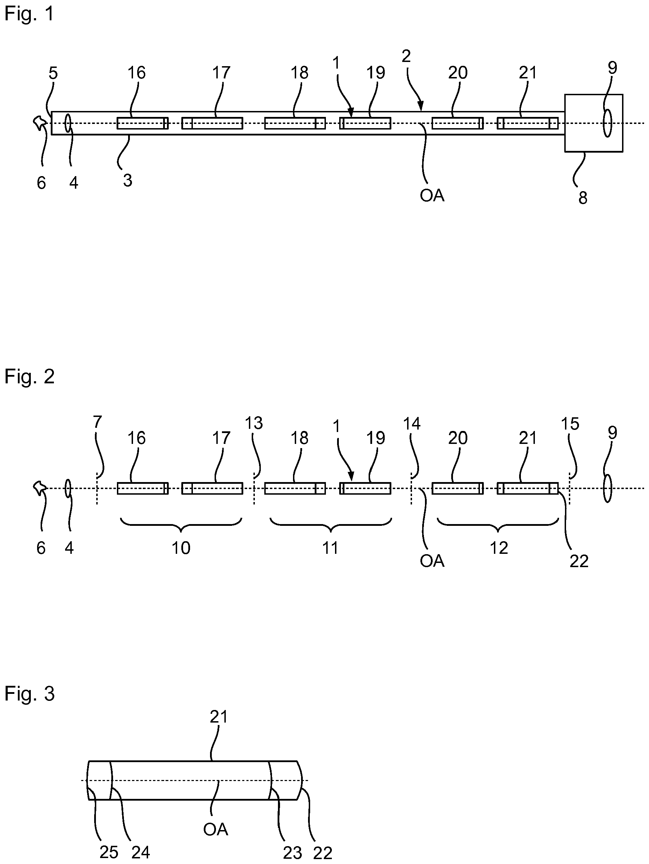

Bei der in Fig. 1 gezeigten Ausführungsform ist das erfindungsgemäße Stablinsensystem 1 in einem schematisch dargestellten Endoskop 2 gezeigt, wobei das Stablinsensystem 1 in einem starren Schaft 3 des Endoskops 2 angeordnet ist.

In dem Schaft 3 ist ferner ein schematisch dargestelltes Objektiv 4 angeordnet, das ein vor dem distalen Ende 5 des Schaftes 3 befindliches Objekt 6 in eine erste Zwischenbildebene 7, die auch als distale Zwischenbildebene 7 bezeichnet werden kann, abbildet (Fig. 2). Ferner umfasst das Endoskop 2 ein Hauptteil 8, in dem eine weitere Optik angeordnet sein kann, wie z.B. das schematisch dargestellte Okular 9.

Das erfindungsgemäße Stablinsensystem 1 weist eine erste, eine zweite und eine dritte Umkehrstufe 10, 11, 12 auf, die hintereinander angeordnet sind und jeweils ein Zwischenbild in eine nächste Zwischenbildebene abbilden. So bildet die erste Umkehrstufe 10 das in der ersten Zwischenbildebene 7 liegende Zwischenbild in eine zweite Zwischenbildebene 13 ab. Das zweite Umkehrsystem 11 bildet das in der zweiten Zwischenbildebene 13 liegende Zwischenbild in eine dritte Zwischenbildebene 14 ab. Das dritte Umkehrsystem 12 bildet das Zwischenbild aus der dritten Zwischenbildebene 14 in eine vierte Zwischenbildebene 15 ab, die auch als proximale Zwischenbildebene 15 bezeichnet werden kann. Die drei Umkehrsysteme 10 - 12 sind somit so hintereinander angeordnet, dass ein in der distalen Zwischenbildebene 7 liegendes Zwischenbild (über die jeweils nächste Zwischenbildebene 13 und 14) in die proximale Zwischenbildebene 15 abgebildet wird. Nachdem jede Umkehrstufe 10 - 12 bei der Abbildung des Zwischenbildes ein umgekehrtes Zwischenbild erzeugt und eine ungerade Anzahl von Umkehrstufen 10 - 12 vorgesehen sind, wird das in der distalen Zwischenbildebene 7 liegende Zwischenbild des Objektes 6 als umgekehrtes Zwischenbild in die proximale Zwischenbildebene 15 abgebildet. Das Stablinsensystem 1 kann daher auch als Umkehrsystem 1 bezeichnet werden.

Dem Stablinsensystem 1 ist das Okular 9 nachgeordnet, so dass ein Benutzer mittels des Okulars 9 das in der proximalen Zwischenbildebene 15 abgebildete Zwischenbild wahrnehmen kann. Natürlich ist es auch möglich, statt des Okulars 9 eine andere Optik vorzusehen, um z. B. das in der proximalen Zwischenbildebene 15 liegende Zwischenbild mittels einer (nicht gezeigten) Kamera aufzunehmen und über ein (nicht gezeigtes) Ausgabesystem oder Anzeigesystem darzustellen.

Das Stablinsensystem 1 ist als unsymmetrisches Stablinsensystem 1 ausgebildet. Des Weiteren sind bei dem hier beschriebenen Ausführungsbeispiel alle drei Umkehrstufen 10 - 12 auch jeweils unsymmetrisch ausgebildet.

Durch die unsymmetrische Ausbildung des Stablinsensystems 1 kann beispielsweise der Farblängsfehler besser korrigiert werden im Vergleich zu symmetrischen Stablinsensystemen.

Ferner kann das Stablinsensystem 1 so ausgelegt sein, dass eine gewünschte Bildgröße in der proximalen Zwischenbildebene 15 erreicht wird. Auch können Telezentriecharakteristiken oder die Lage der Austrittspupille entlang der optischen Achse OA des Stablinsensystems 1 eingestellt werden.

Dadurch, dass das Stablinsensystem 1 im Vergleich zu den herkömmlichen Stablinsensystemen besser auskorrigiert werden kann, kann z.B. der Korrekturaufwand für das Objektiv 4 und/oder das Okular 9 geringer ausfallen. So ist insbesondere die Verzeichnung des gesamten abbildenden Systems aufgrund des unsymmetrischen Stablinsensystems verbessert.

Die unsymmetrische Ausbildung der Umkehrstufen 10 - 12 wird dadurch realisiert, dass pro Umkehrstufe 10 - 12 jeweils zwei Stablinsen 16, 17; 18, 19 und 20, 21 vorgesehen sind, die unterschiedlich bzw. nicht vom gleichen Typ sind. Insbesondere sind bei dem beschrieben Stablinsensystem drei verschieden ausgebildete Stablinsen vorgesehen, die als Typ A, Typ B und Typ C bezeichnet werden können. Die Stablinsen des gleichen Typs zeichnen sich dadurch aus, dass ihre optischen Parameter gleich sind. Das bedeutet, dass sie gleiche Materialien, Abmessungen, Krümmung von Grenzflächen aufweisen. So sind die Stablinsen 16, 19 und 20 vom Typ A, wobei die Stablinsen 16 und 20 die gleiche Orientierung aufweisen (also gleichsinnig angeordnet sind) und die Stablinse 19 zu den Stablinsen 16 und 20 um 180° gedreht angeordnet ist (also gegensinnig angeordnet sind). Die Stablinsen 17 und 18 sind vom Typ B und sind relativ zueinander um 180° gedreht angeordnet. Die Stablinse 21 ist vom Typ C.

Somit weist das Stablinsensystem 1 folgende Reihenfolge der Stablinsen auf:
A-B-B-A-A-C.

Die erste Umkehrstufe 10 weist einen Vergrößerungsfaktor von größer als 1 und die zweite Umkehrstufe 11 weist einen Vergrößerungsfaktor von kleiner als 1 auf. Die Kombination der ersten und zweiten Umkehrstufe 10 und 11 zusammen weist einen Vergrößerungsfaktor bzw. -maßstab von 1 auf, so dass das in der ersten Zwischenbildebene 7 liegende Zwischenbild als 1:1 -Abbildung in die dritte Zwischenbildebene 14 abgebildet wird. Die erste und zweite Umkehrstufe 10, 11 sind zueinander symmetrisch (relativ zur zweiten Zwischenbildebene 13) angeordnet.

Die dritte Umkehrstufe 12 kann einen Vergrößerungsmaßstab aufweisen, der beispielsweise im Bereich von 0,7 - 1,3 liegt.

Die Darstellungen der Umkehrstufen 10-12 sind rein schematisch. Tatsächlich sind die Grenzflächen der einzelnen Stablinsen 16-21 bevorzugt gekrümmt. Bei der hier beschriebenen Ausführungsform sind die Stablinsen 16 - 20 jeweils zweiteilig ausgebildet, wobei die beiden Teile aus unterschiedlichen Materialien gebildet sind. Die Stablinse 21 ist dreiteilig ausgebildet und kann zwei oder auch drei unterschiedliche Materialien aufweisen.

Besonders bevorzugt ist es, die der proximalen Zwischenbildebene 15 zugewandte Grenzfläche 22 als asphärische Fläche auszubilden. Die asphärische Fläche kann dabei rotationssymmetrisch sein oder auch keine Rotationssymmetrie aufweisen. Insbesondere kann sie in den beiden Hauptschnitten zwei unterschiedliche Krümmungen aufweisen.

Wie der vergrößerten schematischen Darstellung in Fig. 3 entnommen werden kann, sind alle Grenzflächen bzw. Materialgrenzflächen 22, 23, 24 und 25 der Stablinse 21 gekrümmt ausgebildet.

## Patentansprüche

1. Stablinsensystem für ein Endoskop, bei dem
das Stablinsensystem (1) eine optische Achse (OA) und mindestens eine Umkehrstufe (10, 11, 12) aufweist, um ein in einer distalen Zwischenbildebene (7) liegendes Zwischenbild in eine proximale Zwischenbildebene (15) abzubilden,
wobei jede Umkehrstufe (10 - 12) mindestens eine Stablinse (16 - 21) aufweist und ein Zwischenbild in eine nächste Zwischenbildebene abbildet,
und wobei das Stablinsensystem (1) als unsymmetrisches Stablinsensystem (1) ausgebildet ist und mindestens eine der Umkehrstufen (10-12) als unsymmetrische Umkehrstufe (10 -12) ausgebildet ist,
wobei eine unsymmetrische Ausbildung bedeutet, dass relativ zu keiner Ebene senkrecht zur optischen Achse (OA) eine Symmetrie hinsichtlich Materialien, Abmessungen und Krümmung von Grenzflächen vorliegt,
wobei drei hintereinander angeordnete Umkehrstufen (10 -12) vorgesehen sind,
**dadurch gekennzeichnet, dass**
alle Umkehrstufen eine erste Stablinse eines ersten Typs, genau zwei der drei Umkehrstufen eine zweite Stablinse eines zweiten Typs und eine dritte der drei Umkehrstufen eine dritte Stablinse eines dritten Typs aufweist,
wobei die dritte Umkehrstufe eine Stablinse des zweiten Typs nicht aufweist und nur die dritte Umkehrstufe die dritte Stablinse aufweist,
wobei die Stablinsen des ersten, zweiten und dritten Typs unterschiedlich sind und Stablinsen des gleichen Typs gleiche Materialien, Abmessungen und Krümmung von Grenzflächen aufweisen,
und wobei zwei der Umkehrstufen (10, 11) relativ zu der zwischen den beiden Umkehrstufen (10, 11) liegenden Zwischenbildebene (13) zueinander hinsichtlich Materialien, Abmessungen und Krümmung von Grenzflächen symmetrisch angeordnet sind.

2. Stablinsensystem nach Anspruch 1, bei dem jede Umkehrstufe (10, 11, 12) genau zwei Stablinsen (16-21) aufweist.

3. Stablinsensystem nach Anspruch 1 oder 2, bei dem mindestens zwei Umkehrstufen (10-12) als unsymmetrische Umkehrstufen (10-12) ausgebildet sind.

4. Stablinsensystem nach einem der obigen Ansprüche, bei dem alle Umkehrstufen (10-12) als unsymmetrische Umkehrstufen (10 -12) ausgebildet sind.

5. Stablinsensystem nach einem der obigen Ansprüche, bei dem eine erste Umkehrstufe (10), die am nächsten an der distalen Zwischenbildebene (7) liegt, einen Abbildungsmaßstab von größer als 1 aufweist.

6. Stablinsensystem nach Anspruch 5, bei dem eine zweite Umkehrstufe (11), die am nächsten zur ersten Umkehrstufe (10) liegt, einen Abbildungsmaßstab von kleiner als 1 aufweist.

7. Stablinsensystem nach Anspruch 6, bei dem die erste und zweite Umkehrstufe (10, 11) zusammen einen Abbildungsmaßstab von 1 aufweisen.

8. Stablinsensystem nach Anspruch 7, bei dem eine dritte Umkehrstufe einen Abbildungsmaßstab aufweist, der im Bereich von 0,7 bis 1,3 liegt.

9. Stablinsensystemnach einem der obigen Ansprüche, bei dem eine der Umkehrstufen (10-12) eine einer der Zwischenbildebenen (7, 13, 14, 15) zugewandte gekrümmte Grenzfläche (22) aufweist, die asphärisch gekrümmt ist.

10. Stablinsensystem nach einem der obigen Ansprüche, bei dem die Umkehrstufen (10 - 12) jeweils zumindest zwei Stablinsen (16, 17; 18, 19, 20, 21) aufweisen, wobei jede der Umkehrstufen (10 - 12) zwei verschiedene Stablinsentypen (16, 17; 18, 19; 20, 21) aufweist.

11. Stablinsensystem nach einem der obigen Ansprüche, bei dem alle Grenzflächen der Stablinsen (16-21) gekrümmt ausgebildet sind.

12. Stablinsensystem nach einem der obigen Ansprüche, bei dem eine der Stablinsen (16 - 21) aus zumindest zwei Teilen aufgebaut ist.

13. Stablinsensystem nach einem der obigen Ansprüche, bei dem das Stablinsensystem einen Abbildungsmaßstab im Bereich von 0,5 bis 2 aufweist.

14. Endoskop mit einem Stablinsensystem (1) nach einem der obigen Ansprüche.

## Claims

1. Rod lens system for an endoscope, in which
the rod lens system (1) has an optical axis (OA) and at least one reversal stage (10, 11, 12), in order to project an intermediate image lying in a distal intermediate image plane (7) into a proximal intermediate image plane (15),
wherein each reversal stage (10- 12) has at least one rod lens (16 - 21) and projects an intermediate image into a next intermediate image plane,
and wherein the rod lens system (1) is formed as an asymmetrical rod lens system (1) and at least one of the reversal stages (10 - 12) is formed as an asymmetrical reversal stage (10 - 12),
wherein an asymmetrical formation means that a symmetry relative to no plane perpendicular to the optical axis (OA) is present in respect of materials, dimensions and curvature of boundary surfaces, wherein three reversal stages (10 - 12) arranged one behind the other are provided,
**characterized in that**
all reversal stages have a first rod lens of a first type, exactly two of the three reversal stages have a second rod lens of a second type and a third of the three reversal stages has a third rod lens of a third type,
wherein the third reversal stage does not have a rod lens of the second type and only the third reversal stage has the third rod lens,
wherein the rod lenses of the first, second and third type are different and rod lenses of the same type have the same materials, dimensions and curvature of boundary surfaces,
and wherein two of the reverse stages (10, 11) are arranged symmetrically to one another relative to the intermediate image plane (13) arranged between both reversal stages (10, 11) in respect of materials, dimensions and curvature of boundary surfaces.

2. Rod lens system according to claim 1, in which each reversal stage (10, 11, 12) has exactly two rod lenses (16-21).

3. Rod lens system according to claim 1 or 2, in which at least two reversal stages (10 - 12) are formed as asymmetrical reversal stages (10 - 12).

4. Rod lens system according to one of the above claims, in which all reversal stages (10 - 12) are formed as asymmetrical reversal stages (10 - 12).

5. Rod lens system according to one of the above claims, in which a first reversal stage (10), which lies closest to the distal intermediate image plane (7), has a magnification factor greater than 1.

6. Rod lens system according to claim 5, in which a second reversal stage (11), which lies closest to the first reversal stage (10), has a magnification factor smaller than 1.

7. Rod lens system according to claim 6, in which the first and second reversal stage (10, 11) together have a magnification factor of 1.

8. Rod lens system according to claim 7, in which a third reversal stage has a magnification factor in the region of 0.7 to 1.3.

9. Rod lens system according to one of the above claims, in which one of the reversal stages (10 - 12) has a curved boundary surface (22), facing one of the intermediate image planes (7, 13, 14, 15), which is aspherically curved.

10. Rod lens system according to one of the above claims, in which the reversal stages (10 - 12) each have at least two rod lenses (16, 17; 18, 19, 20, 21), wherein each of the reversal stages (10 - 12) has two different rod lens types (16, 17; 18, 19; 20, 21).

11. Rod lens system according to one of the above claims, in which all boundary surfaces of the rod lenses (16-21) are formed curved.

12. Rod lens system according to one of the above claims, in which one of the rod lenses (16 - 21) is constructed from at least two parts.

13. Rod lens system according to one of the above claims, in which the rod lens system has a magnification factor in the region of 0.5 to 2.

14. Endoscope with a rod lens system (1) according to one of the above claims.

## Revendications

1. Système de lentilles tiges pour un endoscope, le système de lentilles tiges (1) possédant un axe optique (OA) et au moins un étage d'inversion (10, 11, 12) en vue de représenter dans un plan d'image intermédiaire proximal (15) une image intermédiaire qui se trouve dans un plan d'image intermédiaire distal (7),
chaque étage d'inversion (10 - 12) possédant au moins une lentille tige (16 - 21) et représentant une image intermédiaire dans un plan d'image intermédiaire suivant, et le système de lentilles tiges (1) étant réalisé sous la forme d'un système de lentilles tiges (1) dissymétrique et au moins l'un des étages d'inversion (10 - 12) étant réalisé sous la forme d'un étage d'inversion (10 - 12) dissymétrique,
une représentation dissymétrique voulant dire qu'aucune symétrie du point de vue des matériaux, des dimensions et de la courbure d'interfaces n'existe par rapport à un plan perpendiculaire à l'axe optique (OA),
trois étages d'inversion (10 - 12) disposés les uns derrière les autres étant présents,
**caractérisé en ce que**
tous les étages d'inversion possèdent une première lentille tige d'un premier type, exactement deux des trois étages d'inversion possèdent une deuxième lentille tige d'un deuxième type et un troisième des trois étages d'inversion possède une troisième lentille tige d'un troisième type,
le troisième étage d'inversion ne possédant pas une lentille tige du deuxième type et seul le troisième étage d'inversion possédant la troisième lentille tige,
les lentilles tiges du premier, du deuxième et du troisième type étant différentes et les lentilles tiges du même type possédant les mêmes matériaux, dimensions et la même courbure des interfaces,
et deux des étages d'inversion (10, 11) étant disposés symétriquement l'un à l'autre du point de vue des matériaux, des dimensions et de la courbure des interfaces par rapport au plan d'image intermédiaire (13) qui se trouve entre les deux étages d'inversion (10, 11).

2. Système de lentilles tiges selon la revendication 1, dans lequel chaque étage d'inversion (10, 11, 12) possède exactement deux lentilles tiges (16 - 21) .

3. Système de lentilles tiges selon la revendication 1 ou 2, dans lequel au moins deux étages d'inversion (10 - 12) sont réalisés sous la forme d'étages d'inversion (10 - 12) dissymétriques.

4. Système de lentilles tiges selon l'une des revendications précédentes, dans lequel tous les étages d'inversion (10 - 12) sont réalisés sous la forme d'étages d'inversion (10 - 12) dissymétriques.

5. Système de lentilles tiges selon l'une des revendications précédentes, dans lequel un premier étage d'inversion (10), qui se trouve le plus proche du plan d'image intermédiaire distal (7), possède une échelle de représentation supérieure à 1.

6. Système de lentilles tiges selon la revendication 5, dans lequel un deuxième étage d'inversion (11), qui se trouve le plus proche du premier étage d'inversion (10), possède une échelle de représentation inférieure à 1.

7. Système de lentilles tiges selon la revendication 6, dans lequel le premier et le deuxième étage d'inversion (10, 11) possèdent ensemble une échelle de représentation égale à 1.

8. Système de lentilles tiges selon la revendication 7, dans lequel un troisième étage d'inversion possède une échelle de représentation qui est comprise dans la plage de 0,7 à 1,3.

9. Système de lentilles tiges selon l'une des revendications précédentes, dans lequel l'un des étages d'inversion (10 - 12) possède une interface (22) courbe qui fait face à l'un des plans d'image intermédiaire (7, 13, 14, 15), laquelle est de courbure asphérique.

10. Système de lentilles tiges selon l'une des revendications précédentes, dans lequel les étages d'inversion (10 - 12) possèdent respectivement au moins deux lentilles tiges (16, 17 ; 18, 19, 20, 21), chacun des étages d'inversion (10 - 12) possédant deux types de lentille tige (16, 17 ; 18, 19 ; 20, 21) différents.

11. Système de lentilles tiges selon l'une des revendications précédentes, dans lequel toutes les interfaces des lentilles tiges (16 - 21) sont de configuration courbe.

12. Système de lentilles tiges selon l'une des revendications précédentes, dans lequel les lentilles tiges (16 - 21) sont constituées d'au moins deux parties.

13. Système de lentilles tiges selon l'une des revendications précédentes, dans lequel le système de lentilles tiges possède une échelle de représentation dans la plage de 0,5 à 2.

14. Endoscope comprenant un système de lentilles tiges (1) selon l'une des revendications précédentes.
